# EUROPEAN PATENT SPECIFICATION

(11) **EP 0 476 850 B1**
(45) Date of publication and mention of the grant of the patent: **10.04.1996**
(21) Application number: 91307752.5
(22) Date of filing: 22.08.1991
(51) Int. Cl.: G01N 33/04, C12Q 1/30

(54) **Somatic cell counts**
Zählung somatischer Zellen
Comptages de cellules somatiques

(30) Priority: 23.08.1990 GB 9018540
(43) Date of publication of application: 25.03.1992
(73) Proprietor: Diversey Corporation, Mississauga, Ontario L4Z 3S9 (CA)
(72) Inventor: Corby, Michael Peter, Ravenshead, Nottinghamshire (GB)
(74) Representative: Froud, Clive

(56) References cited:
- DE-B- 2 548 219
- GB-A- 2 202 049
- GB-A- 2 220 066
- US-A- 3 926 732
- JOURNAL OF MILK FOOD TECHNOLOGY, vol. 35, no. 11, 1972, W.D. SCHULTZE et al.,pp. 639-645

## Description

This invention relates to somatic cell counts (SCC); more particularly, it relates to the use of SCC in the detection of intramammary infection in dairy cows.

For many years and for a variety of reasons, there has been considerable interest in the detection of intramammary infection in dairy cows. In addition, under planned regulations, producers would benefit financially from low SCC milk. Such infections are known to be reflected in the number of somatic cells in the milk, being a consequence of the inflammatory response of the udder. Numerous means are known for SCC determination, the Coulter Counter Technique being the standard method. In cases of infection, various chemicals and enzyme systems in the milk change and the SCC is high, e.g. greater than 500,000 cells per ml, as opposed to a normal count of, say, from 50,000 to 200,000 cells per ml. These chemical and enzymatic changes have been sought to be used as the basis for rapid "cowside" tests. However, it is not enough for such "on-farm" tests merely to be quick, they must also be reliable and, above all, convenient.

Somatic cells contain the enzyme catalase and a determination thereof may be used as an indication of SCC. Known methods of quantification have proved impractical in this instance. The enzyme catalyses the evolution of oxygen from hydrogen peroxide and attempts at measurement have involved foam height measurement in test tubes, floating discs in wide test tubes and bubble counting. Correlation coefficients, false positive and false negative responses have been so unsatisfactory as to prevent commercialisation of a reliable test. (See, for example, Krishnamurthy, M.K., and Sastry, K.N.V., Livestock Adviser, 12(4), 39-41, 1987; Schultze, W.D., J. Dairy Science, 56(3), 331-336, 1973; Schultze, W.D., et al, J. Milk and Food Technology, 35(11), 639-645, 1972.)

A surprisingly simple method of assessing oxygen evolution from milk mixed with hydrogen peroxide has now been devised and this facilitates an intramammary infection, in particular mastitis, detection technique which is both accurate and convenient. In accordance with the present invention, immediately the milk and the hydrogen peroxide have been mixed, a portion of the sample is introduced into a capillary tube to a pre-determined level. One end of the tube is blocked and the change in level due to oxygen evolution measured. Hence, the desired indication is provided. For convenience, a scale may be provided. Unlike previous unsuccessful attempts, the present method is simple and practical, as well as highly quantifiable. Instead of simply allowing the sample to rise in a glass or plastic capillary in the normal way, for example, a similar effect may be obtained using a syringe.

Accordingly, the present invention provides a method for the determination of the somatic cell content of milk comprising mixing the milk with hydrogen peroxide and measuring the evolution of gas, characterised in that the gas evolution is measured using a capillary tube.

More particularly, immediately after the milk and hydrogen peroxide have been mixed, a portion of the sample is introduced into a capillary tube to a pre-determined level and one end of the tube is blocked, the change in level due to gas evolution being measured. Alternatively, the sample may be drawn into a syringe, the gas evolution being measured using a capillary tybe in place of the needle. A larger volume of sample would be expected to give a quicker response. Generally, the gas evolution is measured after a pre-determined time, 10 minutes for example, but this may vary fairly widely.

In more detail now, cows, whether or not suspected of mastitis, may be tested in accordance with the present invention. As will be appreciated, milk from an infected quarter must be rejected and directed to waste since infected milk may impact on the overall quality of the milk in the storage vat. Each cow may be tested to determine if the milk available from that cow should be accepted for sale, further processing or use. A sample of milk as extracted from each teat of each cow is tested by placing it in a separate test tube. Into this small sample of milk is added substrate for the enzyme catalase to cause the enzymatic release of gas. As appreciated by those skilled in the art, the substrate for the enzyme catalase may be any of a variety of compounds which, when acted upon by the enzyme catalase, release a detectable amount of gas normally in the form of oxygen, hydrogen or carbon dioxide. According to the preferred embodiment of the present invention, the desired substrate is hydrogen peroxide. A suitable amount of hydrogen peroxide is added to the milk sample in the test tube to provide for the necessary evolution of gas proportional to the concentration of catalase in the sample. The higher the concentration of somatic cells in the sample, the higher the concentration of enzyme and hence a greater volume of gas evolved.

Preferably, immediately after the mixing of the hydrogen peroxide with the milk, the test unit is lowered into the sample. Generally, the test unit comprises an extended section of capillary tube. The test unit is inserted into the tube with the first end lowermost to provide for the introduction of the sample into the capillary of the tube.

The capillary tube has a capillary extending centrally and longitudinally of the tube. On the exterior surface of the tube may be a scale consisting of three gradations. The first gradation is provided to indicate the extent of insertion of the tube into the sample. The sample rises in the capillary to at least the level of the sample in the tube and normally above that level due to capillary attraction. The level of liquid in the capillary is therefore aligned with the first gradation before the test is begun. Of course, a separate scale may be provided. Normally, a finger or other means of blocking the second end of the tube is used to block the second end and permit removal of the tube from the sample without loss of liquid from the capillary.

With the test unit removed from the sample, the liquid level in the capillary is at the first gradation. The first gradation may indicate a zero time for a starting level for the liquid. Immediately after removal of the tube from the sample, it is preferable to block off the first end of the tube. This may be accomplished by a suitable stoppering means, such as mastic, "Plasticine" or putty applied to the first end of the tube. The second end remains open to the atmosphere to allow the liquid to rise in the capillary.

The liquid sample in the lower section of the capillary provides a reservoir for the milk sample where the amount of liquid is of a predetermined volume as identified by the first gradation. As the enzyme catalase slowly works on the hydrogen peroxide in the sample, minute oxygen bubbles are formed in the liquid to cause an expansion and hence travel of the liquid towards the open end of the capillary. For a predetermined period of time, the extent of movement of the liquid is indicative of the somatic cell count in the sample. By comparison with other data and perhaps some trial and error, a correlation in the movement of the liquid along the capillary for a known period may be developed relative to the somatic cell count for each sample. By these results, the gradations may be calculated and optionally etched on each tube. Movement of the liquid level up to, but less than the middle gradation indicates a somatic cell count less than a predetermined amount and hence an acceptable level in the milk sample. The farmer may then confidently accept the milk from the suspected cow. However, if the extent of movement of a liquid is beyond this gradation and up to the third gradation of therebeyond, then the somatic cell count in the sample is too high and hence unacceptable. For liquid levels beyond the third gradation, clearly the milk must be rejected. For intermediate liquid levels, the determination is questionable and the cow requires continued monitoring; the farmer should again reject the milk.

It is to be appreciated that the capillary effect may be accomplished by other devices than a normal tube, such as a rectangular test unit. Such a test unit has opposing walls separated by thin members to define a capillary between the opposing plates. Both ends are open. As indicated above, the test unit may also comprise a syringe with a capillary tube attached to its end fitting. After collection of the milk in the test tube and the addition of hydrogen peroxide thereto, the syringe, without the capillary tube, is used to extract a predetermined quantity of sample from the test tube. The outside surface of the syringe may have the normal markings to indicate withdrawal of a predetermined volume of the sample from the test tube. The plunger is then advanced in the syringe until the exact volume is provided in the syringe. The capillary tube, optionally with a coupling device, is then placed on the end fitment such that the liquid level is at the first end of the capillary tube. The second end of the capillary tube remains open.

The catalase in the milk is then allowed to act on the hydrogen peroxide to generate oxygen gas and hence expand the liquid. Due to the larger volume of liquid in the syringe, liquid moves more quickly up the capillary tube. Hence, in a shorter time the liquid level may be read to assess acceptability of the sample as outlined above. The capillary as provided for detecting movement in the liquid level due to generation of gas bubbles in the sample is of a size to magnify or amplify liquid movement so that such movement is detectable to indicate in the manner discussed an acceptance or rejection of a milk sample. It is to be understood that the concentration of hydrogen peroxide or other enzyme substrate used in the test procedure does not cause a violent or vigorous evolution of gas, since this would normally upset the testing procedure because larger bubbles could be developed which result in spewing of liquid from the open end of the capillary tube. It has been found that the use of hydrogen peroxide is particularly suitable in this regard, since the concentration of catalase in the milk, due to the presence of somatic cells, is relatively dilute so as not to cause excessive gas evolution.

It is appreciated that the volumes and predetermined times and size of capillaries are selected to facilitate the geometry and configuration of any selected test unit design. Based on the following Examples, acceptable predetermined times may range from one to fifteen minutes. Based on test results, the test unit may be calibrated preferably by the markings on the test unit of gradations indicating zero time, region of acceptance, optionally a region of possible rejection and a rejection region. Other forms of calibration may be employed, such as mounting the capillary tube on double-sided tape and placing on a scale already provided on a solid support surface, or placing the capillary tube on the solid support surface and then marking the starting point and then measuring the extent of movement and looking to a calibration curve to determine at least qualitatively the SCC value for the milk sample. It is also understood that with the minute sizing of the capillary, the tube need not necessarily remain vertical during the test procedure. It may be laid on its side, since the liquid will not pass through the open end of the capillary unless it is forced to do so by generation of miniature gas bubbles in the liquid. As is appreciated, the sizing of the capillary is such that the gas bubbles as formed remain minute and do not coalesce to form larger bubbles since liquid is in essence confined and furthermore the predetermined period of time during which the test takes place does not permit extensive coalescing of bubbles to form larger bubbles which could to some extent adversely influence the results of the test. In addition, it is understood that, once the sample is introduced to the capillary, the sample remains undisturbed. By undisturbed, it is intended to mean that the test unit is handled in a manner which avoids any rapid movement which would cause liquid arbitrarily to advance along the capillary.

The following is provided by way of further exemplification of the present invention:

Milk samples were taken direct from cows quarters. Sub-samples were sent to the Milk Marketing Board (MMB) Laboratories for SCC by the standard Coulter Counter Technique, this count is exactly the same as that supplied by the MMB to all farmers in England and Wales. A further sub-sample was used for the test in accordance with the present invention.

3.5ml of milk was placed in a test tube of nominal 5ml capacity so that the depth of the liquid is about 35mm. 0.5ml of 20 vol (6%) hydrogen peroxide solution was added, the tube was capped and inverted to mix.

A glass capillary "melting point" tube, O/D 1.8-2.0mm, I/D 1.2mm, length 100mm (open both ends), was placed in the test tube of milk/hydrogen peroxide mixture and the level allowed to rise in the capillary tube, usually settling about 10mm above the level of the liquid in the outer test tube. A finger was placed over the upper end of the capillary tube to stop air ingress and the capillary tube was withdrawn, a column of sample being-held in the capillary tube. The lower end only of this tube was then blocked by pushing the end of the tube into "Plasticine". The tube was wiped of extraneous mixture and placed such that the level of sample in the capillary tube was noted. (For convenience, the tubes were placed horizontally on double-sided adhesive tape on a solid surface). The liquid/air interface was marked as quickly as possible. After 10 minutes, the distance between the original mark and the liquid/air interface in the capillary tube was measured in millimetres.

Results obtained were as follows:-

| **TEST 1** | | | | | |
|---|---|---|---|---|---|
| SAMPLE No | SCC x10³ | MOVEMENT MM 10 MIN | SAMPLE No | SCC x10³ | MOVEMENT MM 10 MIN |
| 1053 | 82 | 0.5 | 1014 | 106 | 0.5 |
| 1049 | 221 | 0.5 | 1022 | 892 | 7.5 |
| 1024 | 977 | 14.5 | 1018 | 6331 | >36 |
| 1029 | 1053 | 13.5 | 1035 | 100 | 0.5 |
| 1032 | 358 | 1.5 | 1037 | 31 | 0.5 |
| 1010 | 1923 | 5.0 | 1039 | 52 | 1.0 |
| 1017 | 335 | 1.5 | 1045 | 232 | 1.0 |
| 1048 | 51 | 0 | 1051 | 119 | 1.5 |
| 1009 | 214 | 0 | 1012 | 1048 | 14.5 |
| 1023 | 973 | 13.5 | 1036 | 168 | 0.7 (5) |
| 1025 | 136 | 1.5 | 1015 | 819 | 5.5 |
| 1050 | 51 | 0.5 | 1027 | 5023 | >35 |
| 1046 | 110 | 0.5 | 1020 | 623 | 7 |
| 1016 | 3024 | 16.0 | 1038 | 1236 | 9 |
| 1026 | 229 | 3.5 | | | |
| 1021 | 114 | 1.7 (5) | | | |
| 1011 | 681 | 12.5 | | | |
| 1030 | 351 | 1.2 (5) | | | |
| 1047 | 78 | 1.0 | | | |
| 1019 | 244 | 1.0 | | | |
| 1042 | 87 | 0.5 | | | |
| 1052 | 100 | 0 | | | |
| 1033 | 2498 | 26 | | | |
| 1040 | 74 | 0.5 | | | |
| 1008 | 1012 | 10.5 | | | |
| 1043 | 420 | 2.0 | | | |
| 1028 | 426 | 2.0 | | | |
| 1044 | 125 | 0.5 | | | |
| 1031 | 121 | 0.2 (5) | | | |
| 1013 | 188 | 3.5 | | | |
| 1041 | 195 | 1.5 | | | |
| 1034 | 227 | 1.0 | | | |

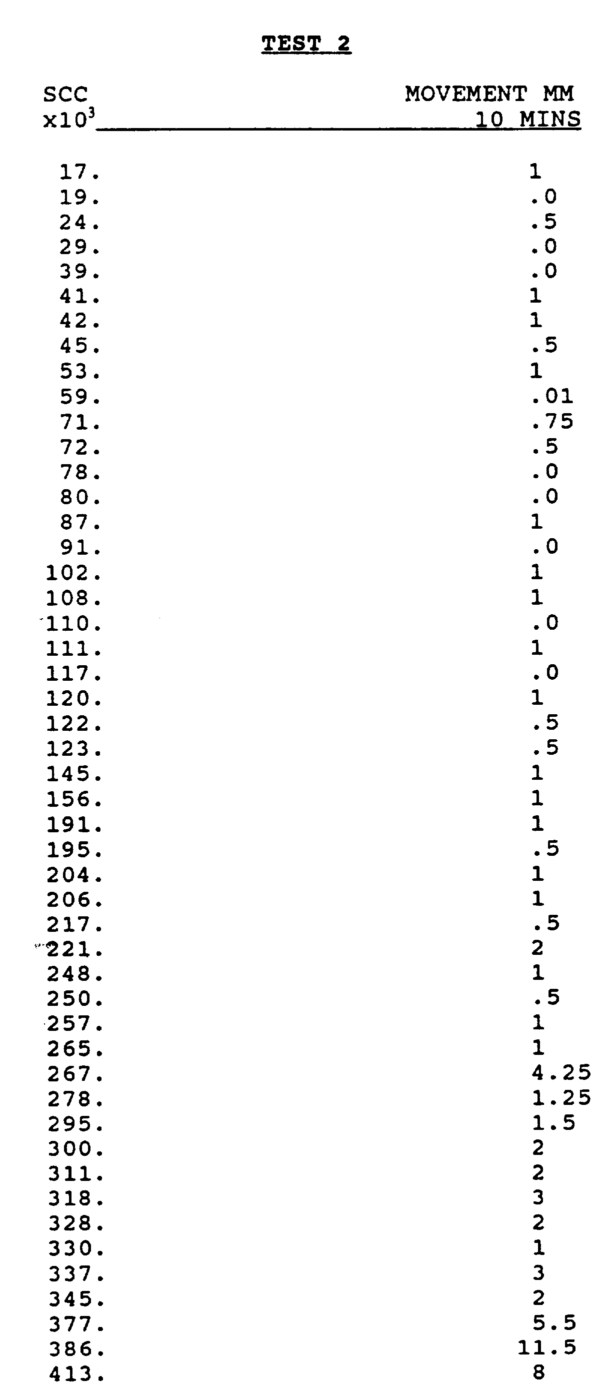

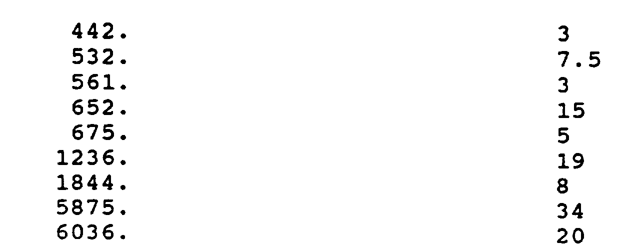

| **TEST 3** | |
|---|---|
| SCC x 10³ | MOVEMENT MM |
| 411 | 1.0 |
| 95 | 1.0 |
| 66 | 0.5 |
| 55 | 0.5 |
| 138 | .25 |
| 422 | 3.0 |
| 71 | 1.0 |
| 49 | 0.5 |
| 1991 | 12.0 |
| 176 | 3.0 |
| 2271 | 34.0 |
| 121 | .0 |
| 196 | .0 |
| 127 | .0 |
| 65 | 1.0 |
| 93 | 1.0 |
| 157 | 1.0 |
| 1168 | 5.0 |
| 131 | 1.0 |
| 1253 | >38.0 |
| 262 | 1.0 |
| 160 | 1.0 |
| 1045 | 3.0 |
| 677 | 27.0 |
| 168 | 0.5 |
| 53 | 0.5 |
| 1882 | 37.0 |
| 75 | 1.0 |
| 66 | 0.5 |

### Analysis of results

(a) Tubes read after 10 minutes.
(b) Movement in mm. >2½ ≡ SCC >500,000-infected.
(c)

| | |
|---|---|
| Total determinations | 128 |
| Total >500,000 SCC | 31 |
| Total false positives | 3 |
| Total false negatives | 3 |
| Specificity | 90.3% |
| Sensitivity | 96.9% |

The data may be re-analysed as follows:
(a) SCC >800,000 - infected.
(b) SCC >300,000 <800,000 - questionable.
(c) SCC <300,000 - uninfected.
(d) Movement >6mm - infected
(e) Movement >2mm <6mm - questionable.
(f) Movement <2mm - uninfected.

### Results

| SCC x 10³ | | 128 determinations Movement mm | | Total cases |
|---|---|---|---|---|
| | <2 | >2 <6 | >6 | |
| <300 | 79 (95%) | 4 (5%) | 0 | 83 |
| >300 <800 | 4 (20%) | 10 (50%) | 6 (30%) | 20 |
| >800 | 0 | 5 (20%) | 20 (80%) | 25 |

As mentioned above, a syringe may be used to provide even greater rapidity and commercial attraction.

35 ml of milk were mixed with 5 ml of hydrogen peroxide. Immediately, the mixture was drawn into a nominal 40 ml hypodermic syringe. The plunger was positioned so that milk/peroxide mixture remained just at the end of the hypodermic. A plastic capillary tube was then placed over the end fitting of the syringe. As oxygen was released, the mixture was forced into the tube and the change in level measured.

| Movement mm | | | | Movement mm | | | |
|---|---|---|---|---|---|---|---|
| 1 min | 2 mins | 3 mins | SCCx10³ | 1 min | 2 mins | 3 mins | SCCx10³ |
| 1 | 2½ | 4 | 1991 | 1½ | 1½ | 1½ | 121 |
| 0 | 0 | 0 | 196 | ½ | 1 | 1 | 66 |
| 4½ | 10 | 17 | 677 | 4 | 8 | 9 | 176 |
| 0 | 0 | 0 | 138 | 1 | 1 | 2 | 482 |
| 0 | 0 | 0 | 168 | 0 | 0 | 0 | 127 |
| 0 | 0 | 0 | 95 | 12 | 25 | 41 | 1253 |
| ½ | 1 | 1½ | 49 | 0 | 0 | 0 | 262 |
| 1 | 1 | 1 | 75 | 14 | 24 | 37 | 2271 |
| 0 | 0 | 0 | 53 | 1 | 1 | 1 | 66 |
| 0 | 1 | 1½ | 93 | 0 | 0 | 0 | 160 |
| 1½ | 2 | 2½ | 1045 | 0 | 0 | 0 | 1168 |
| 6 | 21 | 29 | 1882 | 0 | 0 | 0 | 422 |
| 0 | 0 | 0 | 411 | 1 | 2 | 2½ | 71 |
| 2 | 3 | 3 | 131 | 0 | 0 | 0 | 55 |
| 0 | 0 | 0 | 599 | 2 | 2½ | 3 | 512 |
| 0 | 0 | 0 | 157 | | | | |

As will be appreciated from the foregoing, certain embodiments of the present invention might be defined as follows:
(1) A method for determining an acceptable somatic cell count in a milk sample where somatic cells release catalase in the said milk, the said method comprising:
   (i) mixing the said milk sample with a quantity of a substrate for the said catalase present in the said milk, the said catalase releasing gas by enzymatic action on the said substrate;
   (ii) introducing the said sample to means for providing a longitudinally extending capillary;
   (iii) providing for movement of the said milk sample along the said capillary means due to gas release for a predetermined time; and
   (iv) calibrating extent of movement of the said milk sample to determine an acceptable somatic cell count.
(2) The method (1) wherein the said capillary means has a scale provided thereon or therewith, the said extent of movement of the said milk sample being calibrated against the said scale whereby:
   (i) calibrated extent of movement less than a first designated gradation on the said scale determining an acceptable somatic cell count; and
   (ii) calibrated extent of movement more than a second designated gradation on the said scale determining an unacceptable somatic cell count.
(3) The method (2) wherein the said first and second designated gradations are the same on the said scale.
(4) The method (2) wherein the said scale includes a gradation indicating starting level of the said milk sample.
(5) The method (1) wherein the said substrate is hydrogen peroxide.
(6) The method (5) wherein the said capillary means is a capillary tube.
(7) The method (6) wherein the said step of introducing the said sample to the said capillary tube comprises introducing through a first end of the said capillary tube the said sample a predetermined distance into the said tube and sealing of the said first end.
(8) The method (6) wherein the said step of introducing the said sample to a capillary tube comprises connecting a first end of the said capillary tube to a predetermined volume of the said sample which is adjacent the said first end.
(9) A capillary tube adapted for use in determining an acceptable somatic cell count in a milk sample, the said capillary tube having a sample containing portion and a tube portion with:
   (i) a time zero gradation at a starting liquid level for the said sample;
   (ii) a first gradation where any movement of a milk sample up to the said first gradation indicates an acceptable somatic cell count; and
   (iii) a second gradation beyond the said first gradation where any movement of a milk sample beyond the said first and second gradations indicates an unacceptable somatic cell count.
(10) The capillary tube (9) wherein means for stoppering the said sample containing portion is provided.
(11) The capillary tube (9) wherein the said sample containing portion is a continuation of the said tube portion with the same internal diameter of tube.
(12) A test unit for qualitatively determining somatic cell count in a milk sample, the said test unit comprising:
   (i) a sample reservoir for containing a predetermined quantity of milk admixed with an additive which generates a gas in proportion to quantity of somatic cells in the said milk;
   (ii) means for providing a capillary along which a milk sample may move;
   (iii) means for connecting the said capillary means to the said sample reservoir; and
   (iv) gradations on the said test unit to indicate:
      (a) starting level for a milk sample;
      (b) acceptable levels of somatic cell count; and
      (c) unacceptable levels of somatic cell count.
(13) A method for the determination of the somatic cell count of milk comprising mixing the milk with hydrogen peroxide and measuring the evolution of gas, characterised in that the gas evolution is measured using a capillary tube.
(14) The method (13) wherein, immediately after the milk and hydrogen peroxide have been mixed, a portion of the sample is introduced into a capillary tube to a pre-determined level and one end of the tube is blocked, the change in level due to gas evolution being measured.
(15) The method (13) wherein the sample is drawn into a syringe having an end fitting to fix a tube and gas evolution being measured using a capillary tube fixed onto the said fitting of the said syringe.
(16) The method (13), (14) or (15) wherein the gas evolution is measured after a pre-determined time.

## Claims

1. A method for the determination of the somatic cell content of milk comprising mixing the milk with hydrogen peroxide and measuring the evolution of gas, characterised in that the gas evolution is measured using a capillary tube.

2. A method as claimed in claim 1 wherein, immediately after the milk and hydrogen peroxide have been mixed, a portion of the sample is introduced into a capillary tube to a pre-determined level and one end of the tube is blocked, the change in level due to gas evolution being measured.

3. A method as claimed in claim 1 wherein the sample is drawn into a syringe and gas evolution is measured using a capillary tube fixed in the place of a needle.

4. A method as claimed in any of claims 1 to 3 wherein the gas evolution is measured after a pre-determined time.

5. A kit for the determination of the somatic cell content of milk characterised in that it comprises catalase substrate, sample provision means and capillary means for measuring gas evolution.

6. A kit as claimed in claim 5 wherein the capillary means has thereon or therewith a calibrated scale.

7. A method for determining an acceptable somatic cell count in a milk sample where somatic cells release catalase in the said milk, characterised in that the said method comprises:
(i) mixing the said milk sample with a quantity of a substrate for the said catalase present in the said milk, the said catalase releasing gas by action on the said substrate;
(ii) introducing the said sample to means for providing a longitudinally extending capillary;
(iii) providing for movement of the said milk sample along the said capillary means due to gas release for a predetermined time; and
(iv) calibrating extent of movement of the said milk sample to determine an acceptable somatic cell count.

8. A method as claimed in claim 7 wherein the said capillary means has a scale provided thereon or therewith, the said extent of movement of the said milk sample being calibrated against the said scale whereby:
(i) calibrated extent of movement less than a first designated gradation on the said scale determining an acceptable somatic cell count; and
(ii) calibrated extent of movement more than a second designated gradation on the said scale determining an unacceptable somatic cell count.

9. A method as claimed in claim 8 wherein the said first and second designated gradations are the same on the said scale.

10. A method as claimed in claim 8 wherein the said scale includes a gradation indicating starting level of the said milk sample.

11. A method as claimed in any of claims 7 to 10 wherein the said substrate is hydrogen peroxide.

12. A method as claimed in any of claims 7 to 11 wherein the said capillary means is a capillary tube.

13. A method as claimed in any of claims 7 to 12 wherein the said introduction of the said sample to the said capillary tube comprises introducing through a first end of the capillary tube the said sample a predetermined distance into the said tube and sealing of the said first end.

14. A method as claimed in any of claims 7 to 12 wherein the said introduction of the said sample to a capillary tube comprises connecting a first end of the said capillary tube to a predetermined volume of the said sample which is adjacent the said first end.

15. A capillary tube adapted for use in determining an acceptable somatic cell count in a milk sample in a method according to any one of claims 1 to 14, the said capillary tube being characterised by having a sample containing portion and a tube portion with:
(i) a time zero gradation at a starting liquid level for the said sample;
(ii) a first gradation where any movement of a milk sample up to the said first gradation indicates an acceptable somatic cell count; and
(iii)a second gradation beyond the said first gradation where any movement of a milk sample beyond the said first and second gradations indicates an unacceptable somatic cell count.

16. A capillary tube as claimed in claim 15 wherein means for stoppering the said sample containing portion is provided.

17. A capillary tube as claimed in claim 15 or claim 16 wherein the said sample containing portion is a continuation of the said tube portion with the same internal diameter of tube.

18. A test unit for qualitatively determining somatic cell count in a milk sample in a method according to any one of claims 1 to 14, characterised in that the said test unit comprises:
(i) a sample reservoir for containing a predetermined quantity of milk admixed with an additive which generates a gas in proportion to quantity of somatic cells in the said milk;
(ii) means for providing a capillary along which a milk sample may move;
(iii)means for connecting the said capillary means to the said sample reservoir; and
(iv) gradations on the said test unit to indicate:
(a) starting level for a milk sample;
(b) acceptable levels of somatic cell count; and
(c) unacceptable levels of somatic cell count.

19. A method for determining an acceptable somatic cell count in a milk sample where somatic cells release catalase in the said milk, characterised in that the said method comprises:
(i) mixing the said milk sample with a quantity of hydrogen peroxide which is a substrate for the said catalase present in the said milk, the said substrate releasing minute oxygen bubbles into and along the said milk sample by enzymatic action on the said substrate;
(ii) introducing the said sample of (i) to means for providing a longitudinally extending capillary;
(iii)providing for movement of the said milk sample of (ii) along the said capillary means due to continued formation of minute oxygen bubbles in and along the said milk sample for a predetermined time, such continued formation of minute oxygen bubbles causing an expansion in and movement thereby of all of the said milk sample along the said capillary means;
(iv) calibrating extent of movement of the said milk sample of (iii) as such movement relates to quantity of catalase in the said milk to determine thereby an acceptable somatic cell count.

20. A method as claimed in claim 19 wherein the said acceptable somatic cell count is less than about 300,000.

## Patentansprüche

1. Verfahren zur Bestimmung des Gehalts an somatischen Zellen in Milch, umfassend das Vermischen der Milch mit Wasserstoffperoxid und die Bestimmung der Gasentwicklung, dadurch gekennzeichnet, daß die Gasentwicklung unter Verwendung eines Kapillarrohres bestimmt wird.

2. Verfahren nach Anspruch 1, wobei, sofort nachdem die Milch und das Wasserstoffperoxid vermischt wurden, ein Teil der Probe in ein Kapillarrohr auf ein vorbestimmtes Niveau eingeführt wird und ein Ende des Rohres versperrt wird, wobei die Niveauänderung aufgrund der Gasentwicklung bestimmt wird.

3. Verfahren nach Anspruch 1, wobei die Probe in eine Spritze aufgezogen und die Gasentwicklung unter Verwendung eines anstelle der Nadel befestigten Kapillarrohres bestimmt wird.

4. Verfahren nach einem der Ansprüche 1 bis 3, wobei die Gasentwicklung nach einer vorbestimmten Zeit bestimmt wird.

5. Kit zur Bestimmung des Gehalts an somatischen Zellen in Milch, dadurch gekennzeichnet, daß er ein Katalasesubstrat, eine Probenbereitstellungseinrichtung und eine Kapillareinrichtung zur Bestimmung der Gasentwicklung umfaßt.

6. Kit nach Anspruch 5, wobei auf der Kapillareinrichtung oder mit der Kapillareinrichtung eine kalibrierte Skala vorgesehen ist.

7. Verfahren zur Bestimmung einer zulässigen Anzahl somatischer Zellen in einer Milchprobe, wobei die somatischen Zellen Katalase in die Milch freisetzen, dadurch gekennzeichnet, daß das Verfahren umfaßt:
(i) Vermischen der Milchprobe mit einer Substratmenge für die in der Milch vorliegende Katalase, wobei die Katalase durch Einwirkung auf das Substrat Gas freisetzt;
(ii) Einführen der Probe in eine Einrichtung zur Bereitstellung einer langgestreckten Kapillare;
(iii) Bereitstellen einer Einrichtung zur Bewegung der Milchprobe längs der Kapillare aufgrund der Gasfreisetzung für eine vorbestimmte Zeit; und
(iv) Kalibrierung des Bewegungsgrades der Milchprobe zur Bestimmung einer zulässigen somatischen Zellzahl.

8. Verfahren nach Anspruch 7, wobei auf der Kapillareinrichtung oder mit der Kapillareinrichtung eine Skala vorgesehen ist, wobei der Grad der Bewegung der Milchprobe gegen die Skala kalibriert wird, wodurch:
(i) ein kalbrierter Bewegungsgrad unter einer ersten bestimmten Abstufung auf der Skala eine zulässige somatische Zellzahl angibt; und
(ii) ein kalibrierter Bewegungsgrad über einer zweiten bestimmten Abstufung auf der Skala eine unzulässige somatische Zellzahl angibt.

9. Verfahren nach Anspruch 8, wobei die ersten und zweiten bestimmten Abstufungen auf der Skala identisch sind.

10. Verfahren nach Anspruch 8, wobei die Skala eine Abstufung umfaßt, die das Startniveau der Milchprobe angibt.

11. Verfahren nach einem der Ansprüche 7 bis 10, wobei das Substrat Wasserstoffperoxid ist.

12. Verfahren nach einem der Ansprüche 7 bis 11, wobei die Kapillareinrichtung ein Kapillarrohr ist.

13. Verfahren nach einem der Ansprüche 7 bis 12, wobei die Einführung der Probe in das Kapillarrohr die Einführung der Probe durch ein erstes Ende des Kapillarrohrs eine vorbestimmte Strecke weit in das Rohr und Verschließen des ersten Endes umfaßt.

14. Verfahren nach einem der Ansprüche 7 bis 12, wobei die Einführung der Probe in das Kapillarrohr das Verbinden eines ersten Endes des Kapillarrohres mit einem vorbestimmten Volumen der Probe umfaßt, die an das erste Ende angrenzt.

15. Kapillarrohr, das zur Verwendung bei der Bestimmung einer zulässigen Zellzahl in einer Milchprobe in einem Verfahren nach einem der Ansprüche 1 bis 14 ausgelegt ist, wobei das Kapillarrohr dadurch gekennzeichnet ist, daß es einen die Probe enthaltenden Teil und einen Rohrteil aufweist, mit:
(i) einer Null-Zeiteinteilung am Flüssigkeitsstartniveau für die Probe;
(ii) einer ersten Einteilung, wobei irgendeine Bewegung einer Milchprobe bis zu einer ersten Einteilung eine zulässige somatische Zellzahl anzeigt; und
(iii) eine zweite Einteilung über der ersten Einteilung, wobei eine Bewegung einer Milchprobe über die ersten und zweiten Einteilungen eine unzulässige somatische Zellzahl anzeigt.

16. Kapillarrohr nach Anspruch 15, wobei eine Einrichtung zum Verschließen des die Probe enthaltenden Teils vorgesehen ist.

17. Kapillarrohr nach Anspruch 15 oder Anspruch 16, wobei der die Probe enthaltende Teil eine Fortsetzung des Rohrteils mit dem gleichen Innendurchmesser des Rohrs ist.

18. Testeinheit zur qualitativen Bestimmung der somatischen Zellzahl in einer Milchprobe in einem Verfahren nach einem der Ansprüche 1 bis 14, dadurch gekennzeichnet, daß die Testeinheit umfaßt:
(i) einen Probenspeicher zur Aufnahme einer vorbestimmten Milchmenge, vermischt mit einem Additiv, das im Verhältnis zur Menge an somatischen Zellen in der Milch ein Gas erzeugt;
(ii) eine Einrichtung zur Bereitstellung einer Kapillare, entlang der sich eine Milchprobe bewegen kann;
(iii) eine Einrichtung zur Verbindung der Kapillareinrichtung mit dem Probenspeicher; und
(iv) Einteilungen auf der Testeinheit zum Anzeigen von:
(a) Startniveau für eine Milchprobe;
(b) zulässige Mengen an somatischer Zellzahl; und
(c) unzulässige Mengen an somatischer Zellzahl.

19. Verfahren zur Bestimmung einer zulässigen somatischen Zellzahl in einer Milchprobe, wobei die somatischen Zellen Katalase in die Milch freisetzen, dadurch gekennzeichnet, daß das Verfahren umfaßt:
(i) Vermischen der Milchprobe mit einer Menge an Wasserstoffperoxid, die ein Substrat für die in der Milch vorhandene Katalase darstellt, wobei das Substrat winzige Sauerstoffbläschen in und längs der Milchprobe durch enzymatische Einwirkung auf das Substrat freisetzt;
(ii) Einführen der Probe von (i) in eine Einrichtung zur Bereitstellung einer langgestreckten Kapillare;
(iii) Bereitstellung einer Einrichtung zur Bewegung der Milchprobe aus (ii) längs der Kapillare, wobei die Bewegung auf die fortgesetzte Bildung winziger Sauerstoffbläschen in und längs der Milchprobe für eine vorbestimmte Zeit zurückzuführen ist, und die fortgesetzte Bildung winziger Sauerstoffbläschen eine Ausdehnung in der gesamten Milchprobe und eine Bewegung der Milchprobe längs der Kapillareinrichtung bewirkt;
(iv) Kalibrieren des Umfangs der Bewegung der Milchprobe aus (iii), da diese Bewegung mit der Katalasemenge in der Milch in Beziehung steht, um hierdurch eine zulässige Zahl an somatischen Zellen zu bestimmen.

20. Verfahren nach Anspruch 19, wobei die zulässige Anzahl somatischer Zellen unter etwa 300.000 beträgt.

## Revendications

1. Procédé pour la détermination de la teneur en cellules somatiques du lait consistant à mélanger le lait avec du peroxyde d'hydrogène et à mesurer le dégagement d'un gaz, caractérisé en ce que le dégagement gazeux est mesuré en utilisant un tube capillaire.

2. Procédé selon la revendication 1, dans lequel, immédiatement après avoir mélangé le lait et le peroxyde d'hydrogène, une portion de l'échantillon est introduite dans un tube capillaire à un niveau prédéterminé et une extrémité du tube est bloquée, la modification du niveau due au dégagement gazeux étant mesurée.

3. Procédé selon la revendication 1, dans lequel l'échantillon est prélevé dans une seringue et le dégagement gazeux est mesuré en utilisant un tube capillaire fixé à la place d'une aiguille.

4. Procédé selon l'une des revendications 1 à 3, dans lequel le dégagement gazeux est mesuré après un temps prédéterminé.

5. Trousse pour la détermination de la teneur en cellules somatiques du lait, caractérisée en ce qu'elle comprend un substrat de catalase, un moyen de prélèvement d'échantillon et un moyen capillaire pour mesurer un dégagement gazeux.

6. Trousse selon la revendication 5, dans laquelle le moyen capillaire possède, sur lui ou avec lui, une échelle étalonnée.

7. Procédé pour déterminer un comptage acceptable de cellules somatiques dans un échantillon de lait, où les cellules somatiques libèrent de la catalase dans ledit lait, caractérisé en ce que ledit procédé comprend les étapes consistant:
(i) à mélanger ledit échantillon de lait avec une quantité d'un substrat pour ladite catalase présente dans ledit lait, ladite catalase libérant un gaz par action sur ledit substrat;
(ii) à introduire ledit échantillon dans un moyen procurant un capillaire s'étendant longitudinalement;
(iii) à permettre un déplacement dudit échantillon de lait le long dudit moyen capillaire dû à la libération de gaz pendant un temps prédéterminé; et
(iv) à étalonner la valeur du déplacement dudit échantillon de lait pour déterminer un comptage acceptable de cellules somatiques.

8. Procédé selon la revendication 7, dans lequel ledit moyen capillaire possède une échelle fournie sur lui ou avec lui, ladite valeur de déplacement dudit échantillon de lait étant étalonnée par rapport à ladite échelle, dans lequel:
(i) une valeur étalonnée de déplacement inférieure à une première graduation désignée sur ladite échelle détermine un comptage acceptable de cellules somatiques; et
(ii) une valeur étalonnée de déplacement supérieure à une deuxième graduation désignée sur ladite échelle détermine un comptage inacceptable de cellules somatiques.

9. Procédé selon la revendication 8, dans lequel lesdites première et deuxième graduations désignées sont identiques sur ladite échelle.

10. Procédé selon la revendication 8, dans lequel ladite échelle comprend une graduation indiquant le niveau de départ dudit échantillon de lait.

11. Procédé selon l'une des revendications 7 à 10, dans lequel ledit substrat est du peroxyde d'hydrogène.

12. Procédé selon l'une des revendications 7 à 11, dans lequel ledit moyen capillaire est un tube capillaire.

13. Procédé selon l'une des revendications 7 à 12, dans lequel ladite introduction dudit échantillon dans ledit tube capillaire comprend l'introduction, à travers une première extrémité du tube capillaire, dudit échantillon à une distance prédéterminée dans ledit tube et le scellement de ladite première extrémité.

14. Procédé selon l'une des revendications 7 à 12, dans lequel ladite introduction dudit échantillon dans un tube capillaire comprend la mise en liaison d'une première extrémité dudit tube capillaire avec un volume prédéterminé dudit échantillon, qui est adjacent à ladite première extrémité.

15. Tube capillaire adapté pour une utilisation dans la détermination d'un comptage acceptable de cellules somatiques dans un échantillon de lait par un procédé selon l'une des revendications 1 à 14, ledit tube capillaire étant caractérisé en ce qu'il possède une portion destinée à contenir un échantillon et une portion de tube avec:
(i) une graduation au temps zéro à un niveau de liquide de départ pour ledit échantillon;
(ii) une première graduation pour laquelle tout déplacement d'un échantillon de lait jusqu'à ladite première graduation indique un comptage acceptable de cellules somatiques; et
(iii) une deuxième graduation au-delà de ladite première graduation pour laquelle tout déplacement d'un échantillon de lait au-delà desdites première et deuxième graduations indique un comptage inacceptable de cellules somatiques.

16. Tube capillaire selon la revendication 15, dans lequel un moyen de bouchage de ladite portion destinée à contenir l'échantillon est prévu.

17. Tube capillaire selon la revendication 15 ou 16, dans lequel ladite portion destinée à contenir l'échantillon est une continuation de ladite portion de tube avec le même diamètre interne de tube.

18. Unité de test pour une détermination qualitative du comptage de cellules somatiques dans un échantillon de lait par un procédé selon l'une des revendications 1 à 14, caractérisé en ce que ladite unité de test comprend:
(i) un réservoir d'échantillon pour contenir une quantité prédéterminée de lait mélangée avec un additif qui génère un gaz proportionnellement à la quantité de cellules somatiques dans ledit lait;
(ii) un moyen pour fournir un capillaire le long duquel un échantillon de lait peut se déplacer;
(iii) un moyen pour relier ledit moyen capillaire audit réservoir d'échantillon; et
(iv) des graduations sur ladite unité de test pour indiquer:
(a) le niveau de départ pour un échantillon de lait;
(b) des valeurs acceptables du comptage de cellules somatiques; et
(c) des valeurs inacceptables du comptage de cellules somatiques.

19. Procédé pour déterminer un comptage acceptable de cellules somatiques dans un échantillon de lait où les cellules somatiques libèrent de la catalase dans ledit lait, caractérisé en ce que ledit procédé comprend les étapes consistant:
(i) à mélanger ledit échantillon de lait avec une quantité de peroxyde d'hydrogène, qui est un substrat pour ladite catalase présente dans ledit lait, ledit substrat libérant de minuscules bulles d'oxygène dans et le long dudit échantillon de lait par action enzymatique sur ledit substrat;
(ii) à introduire ledit échantillon de (i) dans un moyen offrant un capillaire s'étendant longitudinalement;
(iii) à permettre le déplacement dudit échantillon de lait de (ii) le long dudit moyen capillaire en raison de la formation continue de bulles minuscules d'oxygène dans et le long dudit échantillon de lait pendant un temps prédéterminé, une telle formation continue de minuscules bulles d'oxygène provoquant une expansion dans ledit moyen capillaire et ainsi un déplacement de l'ensemble dudit échantillon de lait le long dudit moyen capillaire;
(iv) à étalonner l'ampleur du déplacement dudit échantillon de lait de (iii), étant donné qu'un tel déplacement est fonction de la quantité de catalase dans ledit lait, pour déterminer ainsi un comptage acceptable de cellules somatiques.

20. Procédé selon la revendication 19, dans lequel ledit comptage acceptable de cellules somatiques est inférieur à environ 300 000.
